# EUROPEAN PATENT SPECIFICATION

(11) **EP 4 007 627 B1**
(45) Date of publication and mention of the grant of the patent: **14.02.2024**
(21) Application number: 20744077.7
(22) Date of filing: 29.07.2020
(51) Int. Cl.: A61M 11/00, A61M 15/00

(54) **NEBULIZER COMPRISING A MANUALLY ADJUSTABLE USER INTERFACE**
VERNEBLER MIT MANUELL EINSTELLBARER BENUTZERSCHNITTSTELLE
NÉBULISEUR COMPRENANT UNE INTERFACE UTILISATEUR RÉGLABLE MANUELLEMENT

(30) Priority: 02.08.2019 EP 19189889
(43) Date of publication of application: 08.06.2022
(73) Proprietor: Stamford Devices Limited, Dangan, Galway (IE)
(72) Inventor: POWER, Patrick, Galway (IE); CASEY, Micheal, Galway (IE); REDMOND, Anthony, Galway (IE)
(74) Representative: Weldon O'Brien Ltd.
(86) International application number: PCT/EP2020/071390
(87) International publication number: WO 2021/023596

(56) References cited:
- WO-A1-89/06147
- US-A- 5 758 637
- US-A1- 2002 157 662
- US-A1- 2003 150 445
- US-A1- 2013 291 859

## Description

### Introduction

The invention relates to modification of the aerosol output from a nebuliser such as a vibrating mesh nebuliser. The aerosol generator may be a vibrating mesh nebuliser in which a vibratable member is vibrated at ultrasonic frequencies to produce liquid droplets.

Some specific, non-limiting examples of technologies for producing fine liquid droplets is by supplying liquid to an aperture plate having a plurality of tapered apertures extending between a first surface and a second surface thereof and vibrating the aperture plate to eject liquid droplets through the apertures. Such technologies are described generally in U.S. Pat. Nos. US5,164,740; US 5,938,117; US 5,586,550; US 5,758,637; US 6,014,970, US 6,085 740. However, it should be appreciated that the present invention is not limited for use only with such devices.

Various methods of controlling the operation of such nebulisers or aerosol generators are described in US Pat Nos. US6,540,154, US6,845,770, US5,938,117 and US6,546 927.

Other examples are described in WO2010/035251, WO2009/118718, and WO2008/117264 in which a controller has a housing and with user interface buttons and a dial mechanism. There are also status indicators including LEDs. US2013/291859 describes a controller interposed between a host system and a nebulizer, and the controller has a boost circuit, a micro-controller, a drive circuit, and various status indicators. WO2017/066156 and US9352108 also describe systems with controllers linked to aerosol generators. WO89/06147 describes a main controller and not a nebulizer control device as claimed between it and the aerosol generator. US2003/150445 and US2002/157662 describe a main controller controlling a nebulizer The invention is directed towards providing more convenient and versatile aerosol control by al clinician.

### Summary

The invention provides a nebulizer as set out in claim 1. Various optional aspects are set out in the dependent claims.

In one embodiment the controller is adapted to vary the power supply to the aerosol generator. The controller may be adapted to vary droplet size and/or droplet velocity produced by an aerosol generator. In one embodiment the controller is adjustable between a plurality of settings.

In some cases, the controller comprises a manually adjustable slider or a rotatable knob/dial. In one case, the interface is adapted to vary a control signal to the aerosol generator. In one case, the interface is adapted to adjust droplet size and/or droplet velocity produced by an aerosol generator.

In one case, the interface is adjustable between a plurality of settings. In one case, the interface comprises a manually adjustable slider. In one case, the interface comprises a rotatable knob and associated dial. In one case, the interface comprises a thumb-wheel. Preferably, the range is limited to no more than 80% of the maximum aerosol generator range.

In one case, the interface is configured to be linked with a detection device and to automatically adjust aerosol output according to a feed from the detection device.

Preferably, interface is configured to be linked with an end of dose detector, a wet or dry detector, and/or a flow detector.

### Brief Description of the Drawings

The invention will be more clearly understood from the following description thereof, given by way of example only, in which:
Fig. 1 is a diagram showing a nebuliser with a main controller, an aerosol generator, and a manual control device between these two;
Fig. 2 is a diagram illustrating another manual control device;
Fig. 3 illustrates that any of a variety of standard power and signal interfaces such as USB may be linked with the manual control device for connection to a host controller;
Fig. 4 shows manual control components of various embodiments;
Fig. 5 is a diagram showing components of a manual control device of another embodiment, which components allow additional control; and
Figs. 6 and 7 are plots showing signals provided by the device.

### Detailed Description

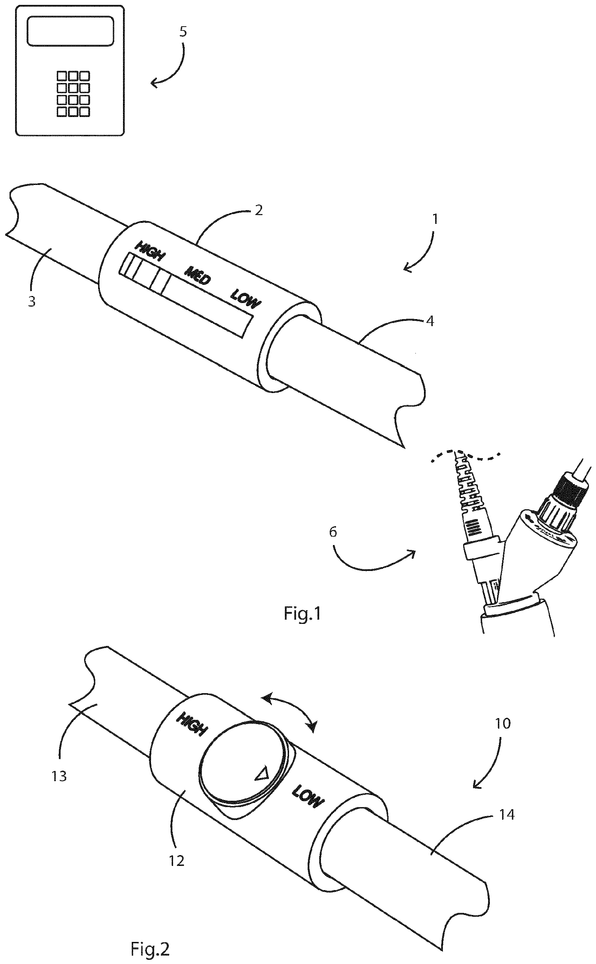

Referring to Fig. 1, a nebuliser manual control device 1 comprises a slider 2, an input cable 3 extending from the interface 3 to a nebulizer main controller 5, and an output cable 4 extending from the interface 2 to an aerosol generator 6.

The manually adjustable user interface 2 is adapted to allow a clinician to manually adjust a signal received from the main controller 5 on the input cable 3 to provide a modified signal on the output cable 4 to manually adjust operation of the aerosol generator 6. As described in more detail below, the slider 2 is an example of a manually adjustable user interface. In this case the manual adjustment is translational or sliding, but it may otherwise be rotational.

The main (or "host") controller 5 to which the cable 3 may be linked has a wall-mounted housing with a user interface and power and control circuits for delivering power and control signals to the aerosol generator 6 via the control device 1.

In this example the aerosol generator 6 which linked to the output cable 4 may be of the type having an aperture plate mounted on a washer support to which is attached a piezo electric actuator. The aerosol generator actuator is controlled by a local control circuit which in one example has a boost circuit with a high frequency, high efficiency DC to DC converter with an integrated power switch capable of providing an output voltage and current profile suitable to drive the nebulizer load. There is also a drive circuit utilizing a series inductor to generate the alternating AC voltage. The drive circuit incorporates a high speed MOSFET driver controlled by a pulse width modulated signal from the microcontroller. There is also a micro-controller with an integrated peripheral module featuring a full speed USB 2.0 compliant interface that can automatically change clock sources and power levels upon connection to a host. The latter provides power and control signals to the aerosol generator head. These signals provide power and control for the vibrating membrane (aperture plate) receiving a liquid to be aerosolised from a feed container. An example of the nebulizer head is described in our previous PCT application WO2012/046220. The controller and the nebulizer head require no more than 500 mA at nominal 5V to generate a desired aerosol.

The nebulizer 6 drive circuit generates an output sine waveform of approximately 100V AC which is fed to the nebulizer head, causing aerosol to be generated. It uses inputs from the micro-controller and the boost circuit to achieve its output. The drive circuit is matched to the impedance of a piezo ceramic element which causes the membrane to vibrate to ensure good energy transfer.

The control device 1 provides for a variable output to be provided to the aerosol generator local controller or head. It uses the power provided on the input cable 3, and varies this power which is passed on to the aerosol generator head. In this example the manually adjustable user interface 2 is a potentiometer, varying a voltage level under manual user control. There is no adjustment at the main controller 5, rather, the adjustment is *en route* to the head in the cable 3/4. The control device is a separate interface to that provided at the source (main controller), and it does not generate a signal itself.

The slider 2 performs the simple task of, under user control, causing the amplitude of the plate vibration to be decreased to reduce output, or the opposite. Altering the resistance provided by the slider 2 varies the power being delivered to the nebuliser, resulting in the flow rate being increased or decreased by increasing or decreasing amplitude of the vibrating mesh.

The cable can be retrofitted to existing main controllers or adapted to a new main controller. The main controller sends its regular signal, the potentiometer of the control device 2 dividing the voltage according to the manual control, and the reduced voltage results in a lower vibration amplitude and slower output.

In one example the cable 4 conducts a DC control signal, the amplitude of which is varied by operation of the user slider 2. This causes the slider 2 to vary the actuation of the aperture plate accordingly. The cable section 4 in this case also has conductors for the AC mains supply to the aerosol generator controller. However, in other examples it is separate and is a stand-alone cable which is easily accessible.

Fig. 2 illustrates another nebuliser manually adjustable control device, 10, which comprises a manually adjustable rotatable knob/dial 12, an input cable 13 and an output cable 14 as described above. In this case the manual control is rotation of the dial 12 to cause an increase or a decrease in power to the head 6.

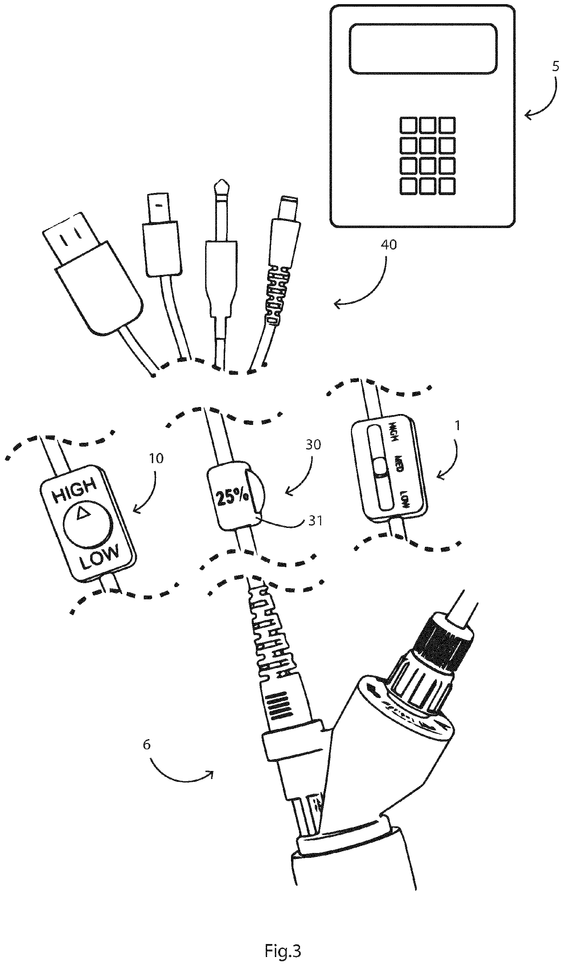

Fig. 3 illustrates that the input cables 3 and 13 may be of any of a variety of standard power and signal types 40 such as USB. The devices 1 and 10 are shown as examples of the invention, and another example shown is a control device 30 with a thumb-wheel manually adjustable user interface 31.

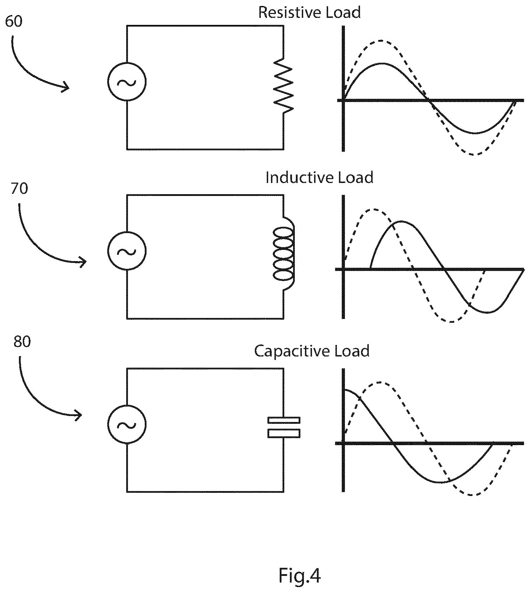

Figs. 1 to 3 show that the control device of the invention may have a manually adjustable user interface which physically moves in either a liner or translational manner (2), or in a rotary manner (12, 31). As shown in Fig. 4, the manual user interface may in various examples cause variation in a resistive load (60), an inductive load (70), or a capacitive load (80). This control is between a host or main controller and the nebulizer head. It will be appreciated that the cable has resistive, capacitive, and inductive properties of its own. Essentially, the manual control device allows one or more of these properties to be varied manually.

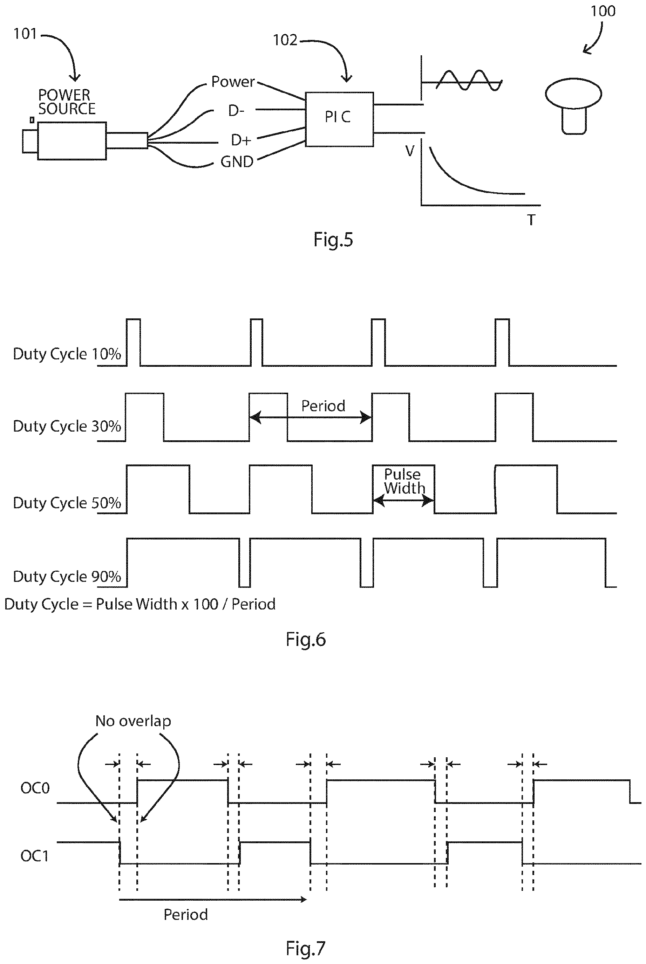

A manually adjustable control device may have a controller such as a PIC microcontroller 102 fed by a power source 101 to perform control of waveforms *en route* to the nebulizer head. This may vary duty cycle as shown in Fig. 6 or phase, as shown in Fig. 7. These parameters may be varied only by the manual interface or additionally by the microcontroller 102 I response to a control signal.

It will be appreciated that the manual control device may for example be used to adjust nebuliser performance in conjunction with drug input feed or for any other reason, operating on the principles of varying resistive and/or capacitive and/or inductive loading and/or phase. Turning/sliding the switch increases/decreases the resistance, inductance or capacitance within the cable.

In the various embodiments the control device or interface provides an in-line, variable potentiometer (or transistor or amplifier) that enables the user to easily and reliably vary the output rate (ml/min), droplet velocity (m/s) and/or droplet size (µm) produced by a vibrating mesh nebuliser as desired. In a preferred embodiment the range of control is limited to adjustment within a limited range which is fixed or is provided by the main controller. This range is preferably above zero, so that the interface cannot be used to shut off operation of the aerosol generator. In general, it is preferred that the range be from about 20% to about 80% of the full output capability of the aerosol generator.

The interface may be used to increase the percentage of drug/saline delivered to the lung.

In the disclosure, the in-line cable that runs from the control device or interface (1, 10, 30) or other embodiment to the nebuliser aerosol generator head incorporates a variable output which is compatible with any of a large number of aerosol controllers in the field.

By enabling the clinician to tailor nebuliser performance to a patient and treatment modality aerosol delivery is improved, resulting in better patient outcomes and increased clinician satisfaction. Decreased aerosol losses in the breathing circuit reduces rain-out accumulation and associated issues.

The in-line, variable interface will allow the clinician to alter/synchronise the output of the nebuliser to match the delivery rate of the saline/medication. This will ensure a more regulated/consistent process of aerosol delivery to the end patient.

In other examples the interface could be, additionally to manual control, electronically controlled and linked to a secondary feature such as devices for detecting end of dose, wet or dry detect, and flow controller (liquid or gas). In these cases, the user interface will automatically regulate the output of the nebuliser depending on the feedback from the above, so that the output of the nebuliser could be rapidly pulsed to alter the output performance of a fixed geometry nebuliser for flow rate and particle size.

The in-line interface could be electronically controlled to calibrate the performance of each vibrating mesh nebuliser used in conjunction with the control module. The nebuliser could be calibrated regarding flow rate and particle size.

Nebulisers can be used as a means to deliver moisture to the airways as an alternative or support to traditional humidification methods. The current issue with this approach is that nebuliser output rates are, in some cases, too high. Enabling the clinician to reduce output enables vibrating mesh nebulisers to be a viable means of delivering moisture to patient airways.

The disclosure is applicable across the spectrum of vibrating mesh aerosol delivery. Its benefits are likely most acute for high-flow delivery and saline delivery for humidification support. The invention allows the clinician to vary output characteristics as desired. For example, reducing the output rate will increase aerosol drug delivery efficiency by reducing aerosol density thus reducing the likelihood of aerosol losses through collision. The aerosol velocity will also be decreased reducing the impact of droplets again reducing aerosol losses. The compromise with reducing output will be an increased nebulisation time. As such in cases where speed of delivery is paramount clinicians may instead choose maximum output rate sacrificing deliver efficiency for increased output.

Practical examples would include an asthmatic receiving treatment in the emergency room where fast delivery would be preferable, and a ventilated patient on a HME (heat and moisture exchanger) where a slow delivery of saline would be best.

The disclosure supports vibrating mesh nebulisers in becoming an alternative/support to traditional means of (hot pot) humidification.

The invention allows clinicians to customise nebuliser performance to their patient and treatment modality. It may be easily retrofitted to an installed base with minimum modification.

The disclosure is not limited to the embodiments hereinbefore described, which may be varied in detail within the scope of the claims. For example, the control device may additionally incorporate a circuit for nebuliser recognition to determine compatibility and to log data about its operation. In general, it may monitor drug feed and regulate output of nebuliser. Also, the control device may synchronise with the drug delivery based on monitoring the electrical parameters of the nebuliser wet/dry cycles, and/or it could log drug delivered and dosing routine.

## Claims

1. A nebulizer comprising:
an aerosol generator (6),
a main controller (5) adapted to drive the aerosol generator (6), and
a nebulizer control device connected by an input cable (3) to the main controller (5) and by an output cable (4) to the aerosol generator (6),
wherein the nebuliser control device (1, 10) comprises:
a manually adjustable user interface (2, 12, 31),
an input cable (3, 13) extending from the interface to the main controller (5), and
an output cable (4, 14) extending from the interface (2, 12, 31) to the aerosol generator (6),
wherein:
the nebulizer control device is adapted to locally, by user manual adjustment of the user interface:
adjust a signal received on the input cable to provide a modified signal on the output cable to adjust operation of an aerosol generator,
vary power supplied to the aerosol generator, and to
adjust a signal to vary the output rate, and to limit the extent of adjustment to a range less than a range from shut-off to maximum output rate.

2. A nebulizer as claimed in claim 1, wherein the manually adjustable user interface is adapted to adjust a signal to vary droplet size and/or droplet velocity produced by an aerosol generator.

3. A nebulizer as claimed in either of claims 1 or 2, wherein the manually adjustable user interface is adjustable between a plurality of settings.

4. A nebulizer as claimed in any of claims 1 to 3 wherein the manually adjustable user interface comprises a manually adjustable slider (2).

5. A nebulizer as claimed in any of claims 1 to 3 wherein the manually adjustable user interface comprises a rotatable knob and associated dial (10).

6. A nebulizer as claimed in any of claims 1 to 3 wherein the manually adjustable user interface comprises a thumb-wheel (31).

7. A nebulizer as claimed in any preceding claim, wherein said range is limited to no more than 80% of the maximum aerosol generator range.

8. A nebulizer as claimed in any preceding claim, wherein the interface is additionally configured to be linked (101, 102) with a detection device and to automatically adjust aerosol output according to a feed from the detection device.

9. A nebulizer as claimed in claim 8, where interface is configured to be linked with an end of dose detector, a wet or dry detector, and/or a flow detector.

10. A method of controlling a nebulizer of any preceding claim, the method comprising steps of the main controller delivering control signals to the aerosol generator via said input cable (3, 13), said manually adjustable user interface (2, 12, 31), and said output cable (4, 14) according to a main controller control output; and said control signals being varied by manual adjustment of said manually adjustable user interface.

## Patentansprüche

1. Vernebler, umfassend:
einen Aerosolerzeuger (6),
eine Hauptsteuereinheit (5), die dazu angepasst ist, den Aerosolerzeuger (6) anzusteuern, und
eine Verneblersteuervorrichtung, die durch ein Eingangskabel (3) mit der Hauptsteuereinheit (5) und durch ein Ausgangskabel (4) mit dem Aerosolerzeuger (6) verbunden ist,
wobei die Verneblersteuervorrichtung (1, 10) Folgendes umfasst:
eine manuell verstellbare Benutzerschnittstelle (2, 12, 31),
ein Eingangskabel (3, 13), das sich von der Schnittstelle zu der Hauptsteuereinheit (5) erstreckt, und
ein Ausgangskabel (4, 14), das sich von der Schnittstelle (2, 12, 31) zu dem Aerosolerzeuger (6) erstreckt,
wobei:
die Verneblersteuervorrichtung dazu angepasst ist, lokal, durch manuelles Verstellen der Benutzerschnittstelle:
ein auf dem Eingangskabel empfangenes Signal zu verstellen, um ein modifiziertes Signal auf dem Ausgangskabel bereitzustellen, um den Betrieb eines Aerosolerzeugers zu verstellen,
dem Aerosolerzeuger zugeführte Leistung zu variieren, und
ein Signal zu verstellen, um die Ausgaberate zu variieren und das Ausmaß der Verstellung auf einen Bereich zu begrenzen, der geringer als ein Bereich vom Abschalten zur maximalen Ausgaberate ist.

2. Vernebler nach Anspruch 1, wobei die manuell verstellbare Benutzerschnittstelle dazu angepasst ist, ein Signal zu verstellen, um die von einem Aerosolerzeuger produzierte Tröpfchengröße und/oder Tröpfchengeschwindigkeit zu variieren.

3. Vernebler nach einem der Ansprüche 1 oder 2, wobei die manuell verstellbare Benutzerschnittstelle zwischen einer Vielzahl von Einstellungen verstellbar ist.

4. Vernebler nach einem der Ansprüche 1 bis 3, wobei die manuell verstellbare Benutzerschnittstelle einen manuell verstellbaren Schieber (2) umfasst.

5. Vernebler nach einem der Ansprüche 1 bis 3, wobei die manuell verstellbare Benutzerschnittstelle einen drehbaren Knopf und eine zugehörige Skalenscheibe (10) umfasst.

6. Vernebler nach einem der Ansprüche 1 bis 3, wobei die manuell verstellbare Benutzerschnittstelle ein Rändelrad (31) umfasst.

7. Vernebler nach einem der vorangehenden Ansprüche, wobei der Bereich auf nicht mehr als 80 % des maximalen Aerosolerzeugerbereichs begrenzt ist.

8. Vernebler nach einem der vorangehenden Ansprüche, wobei die Schnittstelle zusätzlich dazu konfiguriert ist, mit einer Detektorvorrichtung gekoppelt (101, 102) zu werden und die Aerosolausgabe gemäß einer Eingabe von der Detektorvorrichtung automatisch zu verstellen.

9. Vernebler nach Anspruch 8, wobei die Schnittstelle dazu konfiguriert ist, mit einem Dosisendedetektor, einem Nass-/Trockendetektor und/oder einem Strömungsdetektor gekoppelt zu werden.

10. Verfahren zum Steuern eines Verneblers nach einem der vorangehenden Ansprüche, wobei das Verfahren folgende Schritte umfasst: Liefern, durch die Hauptsteuereinheit, von Steuersignalen an den Aerosolerzeuger über das Eingangskabel (3, 13), die manuell verstellbare Benutzerschnittstelle (2, 12, 31) und das Ausgangskabel (4, 14) gemäß eines Hauptsteuereinheit-Steuerungsausgangs; und Variieren der Steuersignale durch manuelles Verstellen der manuell verstellbaren Benutzerschnittstelle.

## Revendications

1. Nébuliseur comportant :
un générateur d'aérosol (6),
un contrôleur principal (5) adapté pour entraîner le générateur d'aérosol (6), et
un dispositif de commande de nébuliseur connecté par un câble d'entrée (3) au contrôleur principal (5) et par un câble de sortie (4) au générateur d'aérosol (6),
dans lequel le dispositif de commande de nébuliseur (1, 10) comporte :
une interface utilisateur ajustable manuellement (2, 12, 31),
un câble d'entrée (3, 13) s'étendant depuis l'interface jusqu'au contrôleur principal (5), et
un câble de sortie (4, 14) s'étendant depuis l'interface (2, 12, 31) jusqu'au générateur d'aérosol (6),
dans lequel :
le dispositif de commande de nébuliseur est adapté pour effectuer localement, par ajustement manuel effectué par l'utilisateur de l'interface utilisateur, les étapes consistant à :
ajuster un signal reçu sur le câble d'entrée pour fournir un signal modifié sur le câble de sortie pour ajuster le fonctionnement d'un générateur d'aérosol,
faire varier la puissance fournie au générateur d'aérosol, et pour effectuer l'étape consistant à :
ajuster un signal pour faire varier la vitesse de sortie, et pour limiter l'étendue de l'ajustement à une plage inférieure à une plage allant de l'arrêt à la vitesse de sortie maximum.

2. Nébuliseur selon la revendication 1, dans lequel l'interface utilisateur ajustable manuellement est adaptée pour ajuster un signal pour faire varier la taille des gouttelettes et/ou la vitesse des gouttelettes produites par un générateur d'aérosol.

3. Nébuliseur selon l'une ou l'autre des revendications 1 ou 2, dans lequel l'interface utilisateur ajustable manuellement est en mesure d'être ajustée parmi une pluralité de réglages.

4. Nébuliseur selon l'une quelconque des revendications 1 à 3, dans lequel l'interface utilisateur ajustable manuellement comporte un curseur manuellement ajustable (2).

5. Nébuliseur selon l'une quelconque des revendications 1 à 3, dans lequel l'interface utilisateur ajustable manuellement comporte un bouton rotatif et un cadran associé (10).

6. Nébuliseur selon l'une quelconque des revendications 1 à 3, dans lequel l'interface utilisateur ajustable manuellement comporte un molette (31).

7. Nébuliseur selon l'une quelconque des revendications précédentes, dans lequel ladite plage est limitée à pas plus de 80 % de la plage maximum du générateur d'aérosol.

8. Nébuliseur selon l'une quelconque des revendications précédentes, dans lequel l'interface est en outre configurée pour être liée (101, 102) à un dispositif de détection et pour ajuster automatiquement la sortie d'aérosol en fonction d'une alimentation en provenance du dispositif de détection.

9. Nébuliseur selon la revendication 8, dans lequel l'interface est configurée pour être liée à une extrémité d'un détecteur de dose, d'un détecteur sec ou humide, et/ou d'un détecteur d'écoulement.

10. Procédé de commande d'un nébuliseur selon l'une quelconque des revendications précédentes, le procédé comportant les étapes suivantes : le contrôleur principal délivre des signaux de commande au générateur d'aérosol par le biais dudit câble d'entrée (3, 13), de ladite interface utilisateur ajustable manuellement (2, 12, 31), et dudit câble de sortie (4, 14) en fonction d'une sortie de commande du contrôleur principal ; et lesdits signaux de commande sont variés par un ajustement manuel de ladite interface utilisateur ajustable manuellement.
